(19) Europäisches Patentamt
European Patent Office
Office européen des brevets

(11) EP 0 792 870 A1

(12) EUROPÄISCHE PATENTANMELDUNG

(43) Veröffentlichungstag:
**03.09.1997 Patentblatt 1997/36**

(21) Anmeldenummer: **97102048.2**

(22) Anmeldetag: **10.02.1997**

(51) Int. Cl.[6]: **C07C 249/12**, C07C 251/34, C07C 251/50, C07C 251/38, C07C 251/52

(84) Benannte Vertragsstaaten:
**CH DE FR GB IT LI NL**

(30) Priorität: **01.03.1996 DE 19607960**

(71) Anmelder: **Hoechst Schering AgrEvo GmbH**
**13509 Berlin (DE)**

(72) Erfinder: **Kuhn, Birgit, Dr.**
**65779 Kelkheim (DE)**

(54) **Verfahren zur Herstellung von Ketonoximether durch Boronsäurekupplung**

(57) Die Erfindung betrifft ein Verfahren zur Herstellung von Ketonoximether der Formel I

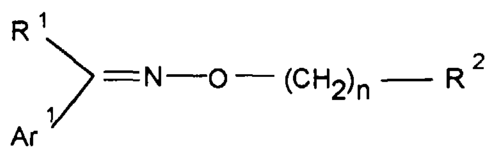

(I),

worin $Ar^1$ einen aromatischen oder heteroaromatischen Ring bedeutet, $R^1$ einen aliphatischen oder carbocyclischen Rest bedeutet, $R^2$ wie $Ar^1$ oder $R^1$ definiert ist und n 0 bis 10 bedeutet, durch Umsetzung von Verbindungen der Formel II

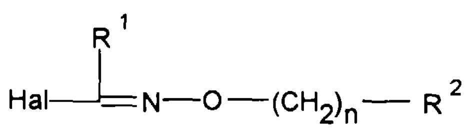

(II),

worin $R^1$, $R^2$ und n wie oben definiert sind, mit den entsprechenden Boronsäuren $Ar^1$-$B(OH)_2$ oder ihren Dimeren oder Trimeren.

Die Erfindung betrifft weiterhin Verbindungen der oben definierten Formel II und Verfahren zu deren Herstellung.

EP 0 792 870 A1

Printed by Rank Xerox (UK) Business Services
2.14.12/3.4

**Beschreibung**

Die vorliegende Erfindung betrifft ein neuartiges Verfahren zur Herstellung von Ketonoximethern der Formel I

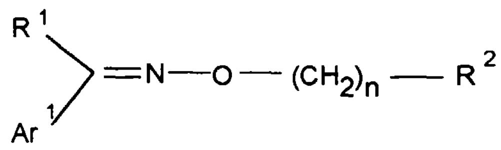

(I),

worin $Ar^1$ einen gegebenenfalls substituierten fünf- oder sechsgliedrigen monocylischen oder anellierten aromatischen oder heteroaromatischen Rest bedeutet, $R^1$ einen gegegenenfalls substituierten Alkyl-, Cycloalkyl-, Alkenyl-, Alkinyl-Rest, bevorzugt Halogenalkyl bedeutet und $R^2$ wie $Ar^1$ oder $R^1$, vorzugsweise wie $Ar^1$ definiert ist. n ist eine ganze Zahle von 0 bis 10, bevorzugt aber 1. Die Verbindungen der Formel I können sowohl als (E)- als auch als (Z)-Isomere oder als (E,Z)-Gemisch oder/und als Salz vorliegen.

Die Erfindung betrifft auch neue Zwischenprodukte der Formel II sowie ein vereinfachtes Verfahren zur Herstellung dieser Zwischenprodukte,

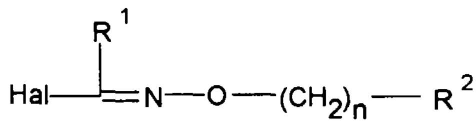

(II),

worin $R^1$, $R^2$ und n wie oben definiert sind und $R^1$ insbesondere Halogenalkyl bedeutet. Hal bedeutet Fluor, Chlor, Brom und Iod, insbesondere aber Chlor und Brom.

Bei dem neuartigen Verfahren zur Herstellung von Verbindungen der Formel I werden Zwischenprodukte der Formel II zusammen mit einer beliebig substituierten Boronsäure der Formel III

$$Ar^1\text{—}B(OH)_2 \qquad (III),$$

worin $Ar^1$ wie oben substituiert ist oder einem Dimeren/Trimeren der Boronsäure der Formel III in Gegenwart einer Palladium-(O)- oder Palladium-(II)-Verbindung als Katalysator und einer organischen und/oder anorganischen Base in einem organischen, evtl. wasserhaltigen Lösungsmittel bzw. Lösungsmittelgemisch bei Temperaturen vorzugsweise zwischen -30°C und + 200°C umgesetzt.

Mit dem erfindungsgemäßen Verfahren werden vorzugsweise Verbindungen der Formel I hergestellt, in der

I.

$Ar^1$

a) $(C_6\text{-}C_{12})$-Aryl oder Heteroaryl mit bis zu 10 C-Atomen bedeutet oder
b) wie unter I.a) definiert ist und bis zu 5 gleiche oder verschiedene Substituenten trägt aus der Reihe

1. $(C_1\text{-}C_6)$-Alkyl,
2. $(C_2\text{-}C_6)$-Alkenyl,
3. $(C_2\text{-}C_6)$-Alkinyl,
4. $(C_3\text{-}C_8)$-Cycloalkyl, gegebenenfalls substituiert mit bis zu 6 gleichen oder verschiedenen Resten aus der Reihe Halogen und $(C_1\text{-}C_4)$-Alkyl,
5. Halogen,
6. $(C_1\text{-}C_6)$-Halogenalkyl,
7. $(C_2\text{-}C_6)$-Halogenalkenyl,
8. $(C_2\text{-}C_6)$-Halogenalkinyl,
9. $(C_1\text{-}C_6)$-Alkoxy-$(C_1\text{-}C_6)$-alkyl,
10. $(C_6\text{-}C_{12})$-Aryl, gegebenenfalls bis zu dreifach substituiert mit gleichen oder verschiedenen Resten aus der Reihe $(C_1\text{-}C_6)$-Alkyl, $(C_1\text{-}C_6)$-Alkoxy, Halogen, $(C_1\text{-}C_6)$-Halogenalkyl und $(C_1\text{-}C_6)$-Halogenalkoxy,
11. Heteroaryl mit bis zu 10 C-Atomen, gegebenenfalls substituiert wie unter I. b) 10.,

12. $(C_6-C_{12})$-Aryl-$(C_1-C_4)$-alkyl, im Arylteil gegebenenfalls substituiert wie unter I. b) 10.,
13. Heteroaryl-$(C_1-C_4)$-alkyl mit bis zu 10 C-Atomen im Heteroarylteil und dort gegebenenfalls substituiert wie unter I. b) 10.,
14. $(C_1-C_6)$-Alkoxy,
15. $(C_2-C_6)$-Alkenyloxy,
16. $(C_2-C_6)$-Alkinyloxy,
17. $(C_3-C_8)$-Cycloalkyloxy, gegebenenfalls substituiert wie unter I. b) 4.,
18. $(C_1-C_6)$-Alkoxy-$(C_1-C_4)$-alkoxy,
19. $(C_6-C_{12})$-Aryloxy, gegebenenfalls substituiert wie unter I. b) 10.,
20. Heteroaryloxy mit bis zu 10 C-Atomen, gegebenenfalls substituiert wie unter I. b) 10.,
21. $(C_1-C_6)$-Halogenalkoxy,
22. $(C_2-C_6)$-Halogenalkenyloxy,
23. $(C_2-C_6)$-Halogenalkinyloxy,
24. $-O-N=CR'_2$, worin R' für gleiche oder verschiedene Reste aus der Reihe Wasserstoff, $(C_1-C_6)$-Alkyl, $(C_3-C_8)$-Cycloalkyl und $(C_6-C_{12})$-Aryl steht,
25. $(C_1-C_6)$-Alkylamino,
26. Di-$(C_1-C_6)$-alkylamino,
27. $(C_3-C_8)$-Cycloalkylamino, gegebenenfalls substituiert wie unter I.b) 4.,
28. $(C_6-C_{12})$-Arylamino, gegebenenfalls substituiert wie unter I. b) 10.,
29. Heteroarylamino mit bis zu 10 C-Atomen, gegebenenfalls substituiert wie unter I. b) 10.,
30. $(C_1-C_6)$-Alkylmercapto,
31. $(C_6-C_{12})$-Arylmercapto,
32. Heteroarylmercapto mit bis zu 10 C-Atomen,
33. $(C_1-C_6)$-Alkylsulfinyl,
34. $(C_6-C_{12})$-Arylsulfinyl,
35. Heteroarylsulfinyl mit bis zu 10 C-Atomen,
36. $(C_1-C_6)$-Alkylsulfonyl,
37. $(C_6-C_{12})$-Arylsulfonyl,
38. Heteroarylsulfonyl mit bis zu 10 C-Atomen,
39. Nitro,
40. Cyano,
41. Cyano-$(C_1-C_6)$-alkyl,
42. $(C_1-C_6)$-Alkoxycarbonyl,
43. $(C_6-C_{12})$-Aryloxycarbonyl und
44. Heteroaryloxycarbonyl mit bis zu 10 C-Atomen im Heteroarylteil, oder
45.

   a) zwei der Substituenten für Methylendioxy stehen,
   b) wobei die Methylendioxygruppe gegebenenfalls mit einem oder zwei gleichen oder verschiedenen Resten aus der Reihe Halogen und $(C_1-C_4)$-Alkyl substituiert ist;

II.

$R^1$

a) $(C_1-C_6)$-Alkyl,
b) $(C_2-C_6)$-Alkenyl,
c) $(C_2-C_6)$-Alkinyl oder
d) $(C_3-C_8)$-Cycloalkyl bedeutet, oder
e) wie unter II. a)-d) definiert ist und bis zu 3 gleiche oder verschiedene Substituenten trägt aus der Reihe

   1. Halogen,
   2. Hydroxy,
   3. $(C_1-C_6)$-Alkoxy,
   4. $(C_1-C_6)$-Alkylmercapto,
   5. $(C_1-C_6)$-Alkylsulfinyl,
   6. $(C_1-C_6)$-Alkylsulfonyl,
   7. Cyano,

8. Nitro und

9. ($C_1$-$C_6$)-Alkoxycarbonyl, oder

f) wie unter II. a)-d) definiert ist und, falls die Anzahl der Wasserstoffatome ≥ 5 ist, diese teilweise oder vollständig durch Halogen ersetzt sind, wobei die Anzahl der Halogenatome ≥ 4 ist;

wobei das unter I. a) und b) definierte Aryl und Heteroaryl auch teilweise hydriert sein kann und darin eine oder zwei $CH_2$-Gruppen durch CO ersetzt sein können.

Alkyl, Alkenyl und Alkinyl können geradkettig oder verzweigt sein. Dies gilt auch für davon abgeleitete Reste wie Alkoxy, Alkylmercapto, Halogenalkyl und Arylalkyl.

Unter Halogenalkyl, Halogenalkenyl und Halogenalkinyl versteht man Alkyl, Alkenyl bzw. Alkinyl, worin ein, mehrere oder alle Wasserstoffatome durch Halogen ersetzt sind. Entsprechendes gilt für davon abgeleitete Reste, wie Halogenalkoxy, Halogenalkenyloxy oder Halogenalkinyloxy.

Halogen bedeutet Fluor, Chlor, Brom oder Iod, vorzugsweise Fluor, Chlor oder Brom.

($C_6$-$C_{12}$)-Aryl steht vorzugsweise für Phenyl und davon abgeleitete Reste wie Naphthyl, Biphenylyl und Indanyl.

Ein Heteroarylrest mit bis zu 10 C-Atomen ist ein vorzugsweise mono- oder bicyclischer Arylrest, in welchem mindestens ein CH durch N ersetzt ist und/oder worin mindestens zwei benachbarte CH-Gruppen durch NH, O und/oder S ersetzt sind. Beispiele solcher Reste sind Thienyl, Benzothienyl, Furyl, Benzofuryl, Pyrrolyl, Imidazolyl, Pyrazolyl, Pyridyl, Pyrazinyl, Pyrimidinyl, Pyridazinyl, Indolyl, Oxazolyl, Isoxazolyl, Thiazolyl und Isothiazolyl.

Sofern die Verbindungen der Formel I Salze bilden können, so sind das insbesondere deren Säureadditionssalze. Säuren, die zur Salzbildung herangezogen werden können, sind anorganische Säuren wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Ölsäure, Methansulfonsäure, Benzolsulfonsäure oder Toluolsulfonsäure.

Besonders bevorzugt werden Verbindungen der Formel I hergestellt, worin

$Ar^1$ Phenyl, Naphthyl, Indanyl, Benzofuryl, Benzothienyl,

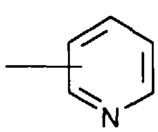 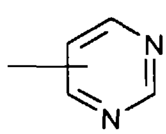 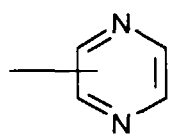 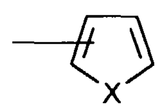

wie Thienyl oder Furanyl oder

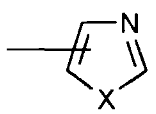 wie Thiazolyl bedeutet,

welche gegebenenfalls wie oben definiert substituiert sind und worin

X für O, S oder $NR^3$ steht und

$R^3$ für Wasserstoff,
($C_1$-$C_6$)-Alkyl,
($C_2$-$C_6$)Alkenyl, vorzugsweise $-CH_2-CH=CH_2$,
($C_2$-$C_6$)-Alkinyl, vorzugsweise $-CH_2-C\equiv CH$,
($C_3$-$C_8$)-Cycloalkyl, gegebenenfalls substituiert mit bis zu 6 gleichen oder verschiedenen Resten aus der Reihe Halogen und ($C_1$-$C_4$)-Alkyl,
($C_1$-$C_6$)-Halogenalkyl,
($C_2$-$C_6$)-Halogenalkenyl,
($C_2$-$C_6$)-Halogenalkinyl,
($C_1$-$C_6$)-Alkoxy-($C_1$-$C_6$)-alkyl,
($C_6$-$C_{12}$)-Aryl-($C_1$-$C_4$)-alkyl,
Heteroaryl-($C_1$-$C_4$)-alkyl mit bis zu 10 C-Atomen im Heteroarylteil,
Cyano-($C_1$-$C_6$)-alkyl,
($C_1$-$C_6$)-Alkoxycarbonyl,

($C_6$-$C_{12}$)-Aryloxycarbonyl oder
Heteroaryloxycarbonyl mit bis zu 10 C-Atomen steht,
insbesondere solche Verbindungen der Formel I, worin

$Ar^1$ einen Rest der Formel

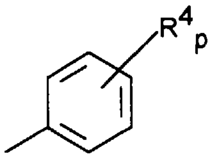
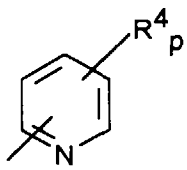
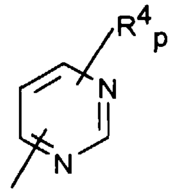
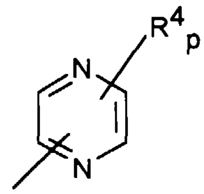

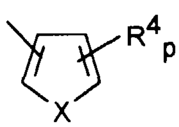
oder
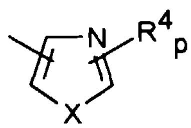

bedeutet,

X wie oben definiert ist,

p eine ganze Zahl von 0 bis 5 ist, und

$R^1$ gleich oder verschieden ist und
Halogen,
($C_1$-$C_6$)-Alkyl,
($C_2$-$C_6$)-Alkenyl,
($C_2$-$C_6$)-Alkinyl,
($C_3$-$C_8$)-Cycloalkyl, gegebenenfalls substituiert mit bis zu 6 gleichen oder verschiedenen Resten aus der Reihe Halogen und ($C_1$-$C_4$)-Alkyl,
($C_1$-$C_6$)-Halogenalkyl,
($C_2$-$C_6$)-Halogenalkenyl,
($C_2$-$C_6$)-Halogenalkinyl,
($C_1$-$C_6$)-Alkoxy-($C_1$-$C_6$)-alkyl,
($C_6$-$C_{12}$)-Aryl, gegebenenfalls bis zu dreifach substituiert mit gleichen oder verschiedenen Resten aus der Reihe Halogen, ($C_1$-$C_6$)-Alkyl, ($C_1$-$C_6$)-Alkoxy, ($C_1$-$C_6$)-Halogenalkyl und ($C_1$-$C_6$)-Halogenalkoxy,
Heteroaryl mit bis zu 10 C-Atomen, gegebenenfalls substituiert wie obengenanntes Aryl,
($C_6$-$C_{12}$)-Aryl-($C_1$-$C_4$)-alkyl, im Arylteil gegebenenfalls substituiert wie obengenanntes Aryl,
Heteroaryl-($C_1$-$C_4$)-alkyl mit bis zu 10 C-Atomen im Heteroarylteil und dort gegebenenfalls substituiert wie obengenanntes Aryl,
($C_1$-$C_6$)-Alkoxy,
($C_2$-$C_6$)-Alkenyloxy,
($C_2$-$C_6$)-Alkinyloxy,
($C_3$-$C_8$)-Cycloalkyloxy,
($C_1$-$C_6$)-Alkoxy-($C_1$-$C_6$)-alkoxy,
($C_6$-$C_{12}$)-Aryloxy, gegebenenfalls substituiert wie obengenanntes Aryl, Heteroaryloxy mit bis zu 10 C-Atomen, gegebenenfalls substituiert wie obengenanntes Aryl,
($C_1$-$C_6$)-Halogenalkoxy,
($C_2$-$C_6$)-Halogenalkenyloxy,
($C_2$-$C_6$)-Halogenalkinyloxy oder
-O-N=CR'$_2$, worin R' gleiche oder verschiedene Reste aus der Reihe Wasserstoff, ($C_1$-$C_6$)-Alkyl, ($C_3$-$C_8$)-Cycloalkyl und ($C_6$-$C_{12}$)-Aryl steht,
bedeutet oder
zwei der Reste für $R^4$ für gegebenenfalls wie unter I. b) 45. b)
substituiertes Methylendioxy stehen,
worin $R^4$ vorzugsweise
($C_1$-$C_6$)-Alkyl,
($C_2$-$C_6$)-Alkenyl,

($C_2$-$C_6$)-Alkinyl,
$C_3$-$C_8$)-Cycloalkyl,
Halogen,
($C_1$-$C_6$)-Halogenalkyl,
($C_2$-$C_6$)-Halogenalkenyl,
($C_2$-$C_6$)-Halogenalkinyl,
gegebenenfalls wie oben substituiertes ($C_6$-$C_{12}$)-Aryl oder Heteroaryl mit bis zu 10 C-Atomen,
($C_1$-$C_6$)-Alkoxy,
($C_2$-$C_6$)-Alkenyloxy,
($C_2$-$C_6$)-Alkinyloxy,
($C_3$-$C_8$)-Cycloalkyloxy,
gegebenenfalls wie oben substituiertes ($C_6$-$C_{12}$)-Aryloxy oder Heteroaryloxy mit bis zu 10 C-Atomen,
($C_1$-$C_6$)-Alkoxy-($C_1$-$C_6$)-alkyl,
($C_1$-$C_6$)-Alkoxy-($C_1$-$C_6$)-alkoxy,
($C_1$-$C_6$)-Halogenalkoxy,
($C_2$-$C_6$)-Halogenalkenyloxy oder
($C_2$-$C_6$)-Halogenalkinyloxy bedeutet,
oder zwei der Reste $R^4$ für gegebenenfalls wie unter I. b) 45. b) substituiertes Methylendioxy stehen,
worin $R^4$ insbesondere ($C_1$-$C_6$)-Halogenalkoxy, wie $OCHF_2$, $OCF_3$, $OCF_2CF_2H$, $OCH_2CF_3$ oder $OCH(CF_3)_2$, ($C_1$-$C_6$)-Alkoxy, Fluor, Chlor, Brom, ($C_1$-$C_6$)-Alkyl, ($C_1$-$C_6$)-Halogenalkenyloxy, ($C_2$-$C_6$)-Halogenalkinyloxy, ($C_1$-$C_6$)-Halogenalkyl, ($C_2$-$C_6$)-Halogenalkenyl oder ($C_2$-$C_6$)-Halogenalkinyl bedeutet.

Die als bevorzugt genannten Reste $R^4$ stehen vorzugsweise in der 4-Position des Phenylrestes, in der dieser entsprechenden Position eines heteroaromatischen Sechsrings oder in analoger Position in einem heterocyclischen Fünfring (s.u.), wobei insbesondere folgende Reste $Ar^1$ bevorzugt sind:

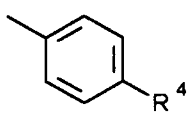 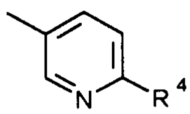 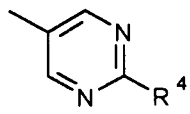 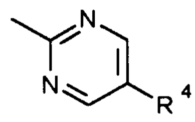 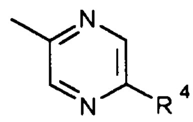

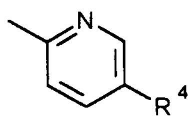 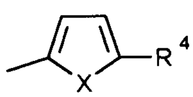 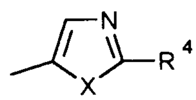 oder 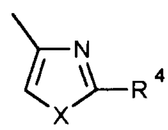

$R^1$ bedeutet vorzugsweise ($C_1$-$C_6$)-Alkyl oder ($C_3$-$C_8$)-Cycloalkyl, welche jeweils teilweise oder vollständig mit Halogen substituiert sein können, insbesondere ($C_1$-$C_6$)-Alkyl, welches mit bis zu 3 Fluor substituiert sein kann, wie $CF_3$, $CF_2H$, oder Isopropyl oder Cyclopropyl.

$R^2$ bedeutet vorzugsweise

A.

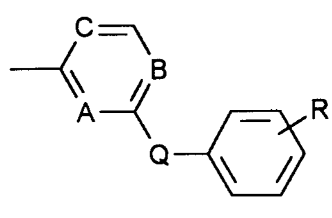

B. a) Pentafluorphenyl,

b) 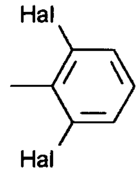

c) 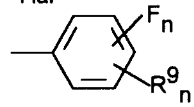

d) 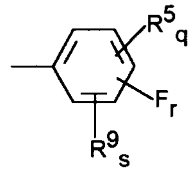

C. a) 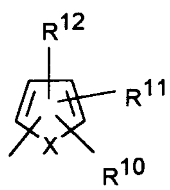

X = O oder S

b) 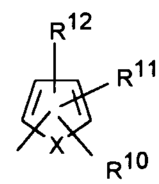

X = $NR^3$

c) 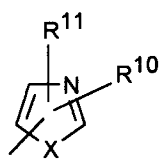

X = O, S oder $NR^3$

d) 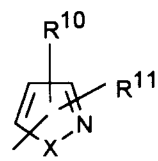

X = O, S oder $NR^3$

D. 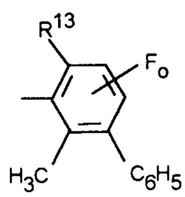

E. 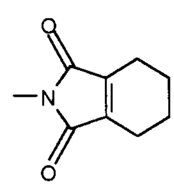

F. a) 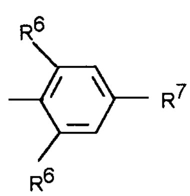

oder

b) 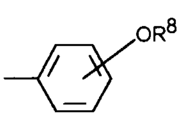

und worin

| | |
|---|---|
| A, B und C | gleich oder verschieden sind und N, CH oder C-Hal bedeuten, |
| Q | O, S, NH oder $CH_2$, vorzugsweise O oder $CH_2$ bedeutet, |
| R | Wasserstoff, Halogen, O-$CH_2$-CH=$CH_2$ oder O-$CH_2$-C≡CH, vorzugsweise Wasserstoff, 4-F, 3-F, 4- |

| | |
|---|---|
| | Cl |
| Hal | für Halogen steht, |
| $R^9$ | Wasserstoff bedeutet oder wie $R^4$ definiert ist, |
| m und n' | gleich oder verschieden sind und jeweils für 1, 2, 3 oder 4 stehen, $m + n' = 5$ ist, |
| $R^5$ | Wasserstoff bedeutet oder wie $R^4$ definiert ist, |
| q, r und s | gleich oder verschieden sind und jeweils für 1, 2 oder 3 stehen, $q + r + s = 5$ ist, |
| $R^3$ | wie oben definiert ist, |
| $R^{10}$, $R^{11}$ und $R^{12}$ | wie oben definiert sind, vorzugsweise $CH_3$, $CF_3$, CN, Phenyl, $CH_2$-CH=$CH_2$, -$CH_2$-C≡CH oder Benzyl bedeuten, |
| $R^{13}$ | Wasserstoff, Fluor oder Methyl bedeutet, |
| o | 0, 1 oder 2 ist, |
| $R^6$ | Wasserstoff, Fluor oder ($C_1$-$C_6$)-Alkyl bedeutet, |
| $R^7$ | Halogenphenoxy, Halogenbenzyl, O-$CH_2$-CH=$CH_2$, O-$CH_2$-C≡CH, $CH_2$-CH=$CH_2$ oder $CH_2$-C≡CH bedeutet und |
| $R^8$ | ($C_2$-$C_6$)-Alkenyl oder ($C_2$-$C_6$)-Halogenalkenyl bedeutet. |

Ist $R^2$ wie oben unter A. definiert, so sind Verbindungen bevorzugt, worin A, B und C nicht alle gleichzeitig N sind, insbesondere solche, worin nur eine der Gruppen A, B oder C N bedeutet. Bevorzugt sind

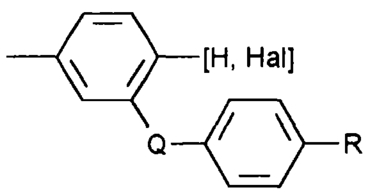

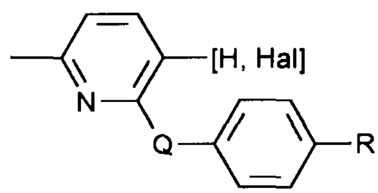

und

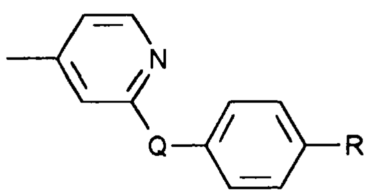

Von den oben unter B. c) definierten $R^2$-Resten ist

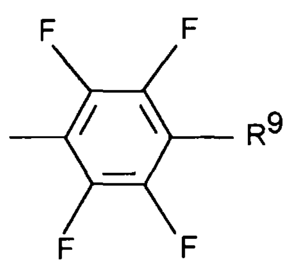

bevorzugt, worin $R^9$ ($C_1$-$C_6$)-Alkyl, insbesondere Methyl, ($C_1$-$C_6$)-Alkoxy, ($C_1$-$C_6$)-Halogenalkoxy oder ($C_1$-$C_6$)-Halogenalkyl bedeutet.

Von den oben unter B. d) definierten $R^2$-Resten sind bevorzugt

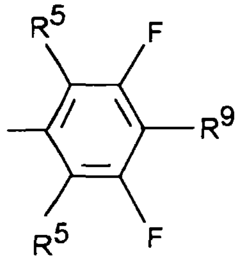

($R^5$ = H, $CH_3$ oder $OCH_3$)

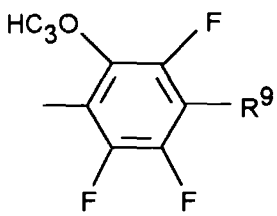

Von den oben unter C. definierten $R^2$-Resten sind bevorzugt

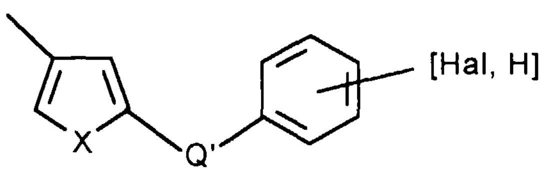

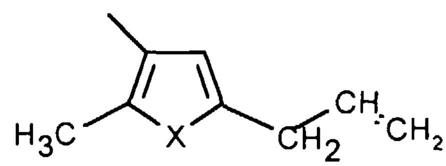

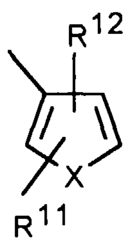

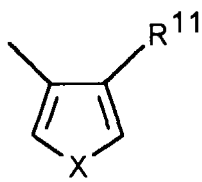

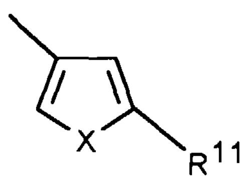

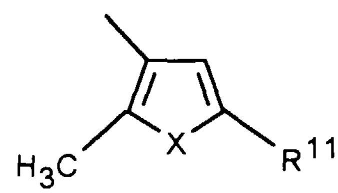

worin
X = $NR^3$, O oder S ist, Q' O oder $CH_2$ bedeutet und $R^{11}$ und $R^{12}$ wie oben definiert sind.

Von den oben unter D. definierten Resten sind jene bevorzugt, worin o = 0 ist, insbesondere solche worin $R^{13}$ Wasserstoff bedeutet.

Es können Diastereomere auftreten. Die Gemische von Diastereomeren können nach gebräuchlichen Methoden, z.B. durch selektive Kristallisation aus geeigneten Lösungsmitteln oder durch Chromatographie, in die Komponenten aufgetrennt werden.

Es sind bereits Verfahren zur Herstellung von Verbindungen der Formel I bekannt (vgl. z.B. Houben-Weyl, Band X/4 S. 55 ff. und E 14 Teil I, S. 367 ff.). Dieser Verbindungstyp ist u. a. in vielen Strukturen aus dem Bereich des Pflanzenschutzes (z. B. WO-A-93/21157, US-A-5194662, EP-A-94398) oder Pharmazeutika (US-A-5231106, US-A-5376680, BE-A-862699) vorhanden.

Die bekannten Verfahren zur Herstellung weisen allerdings einige Nachteile auf. So sind z. B. polare und/oder protische funktionelle Gruppen wie Amino, Hydroxy oder Formyl in den jeweiligen Ausgangsmaterialien nicht erlaubt. Bei der erfindungsgemäßen selektiven Boronsäurekupplung haben wir nun überraschenderweise gefunden (siehe auch A. R. Martin und G. Yang, Acta Chem. Scand. 47 (1993)), daß diese funktionelle Gruppen sich nicht störend auswirken. Bei der Synthese von gängigen Zwischenprodukten zur Herstellung von Oximethern des Typs I, Oxime (IV), fallen diese üblicherweise als Isomerengemisch an (Houben-Weyl, Band X/4 S. 55) und müssen getrennt werden, um möglichst isomerenfreie Verbindungen zu erhalten (Houben-Weyl, Band X/4 S. 291 und Band E 14 a/Teil 1, S. 287 ff.).

Es wurde weiterhin gefunden, daß die für unsere Synthese nötigen Zwischenprodukte vom Typ II überraschenderweise jeweils in hoher Isomerenreinheit (Diastereomerenreinheit) anfallen.

Die Oximroute (Schema 2) stellt die präparativ wohl gebräuchlichste Methode zur Herstellung von Verbindungen des Typs I dar (Houben-Weyl, Band X/4 S. 55).

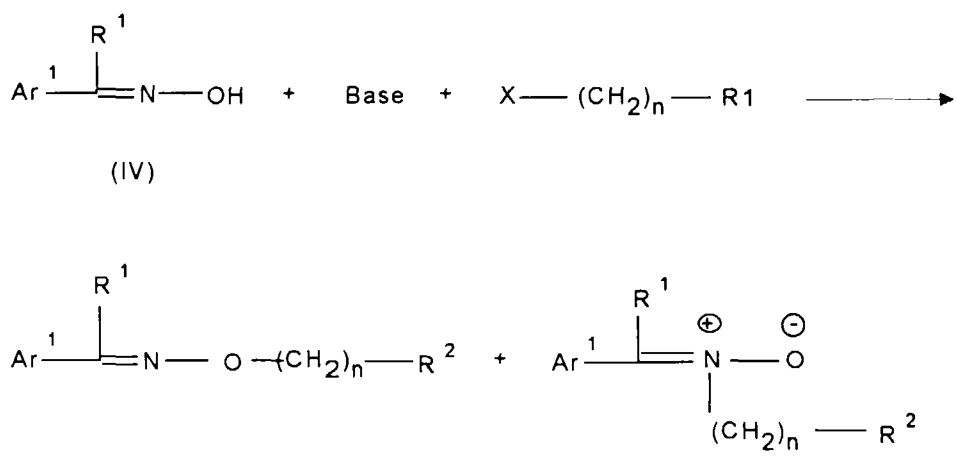

(IV)

(I)

(V)

Schema 2 X = Abgangsgruppe

Allerdings entstehen bei dieser Synthese normalerweise Gemische aus den gewünschten Oximether (I) und dem Nitron (V). Praktisch ausschließlich Oximether der Formel I liefert dort nur die Verwendung von teurem Silberoxid als Base (Houben-Weyl Band X/4 S. 217 ff. und E 14/Teil I S. 367 ff.).

Bei dem erfindungsgemäßen Verfahren erfolgt die Verknüpfung überraschenderweise selektiv, d. h. zwischen der Boronsäure der Formel III und der Halogenverbindung der Formel II nur zu Verbindungen des Formel I unter meist vollständigem Erhalt der Isomerenreinheit der Verbindung der Formel II (Schema 3).

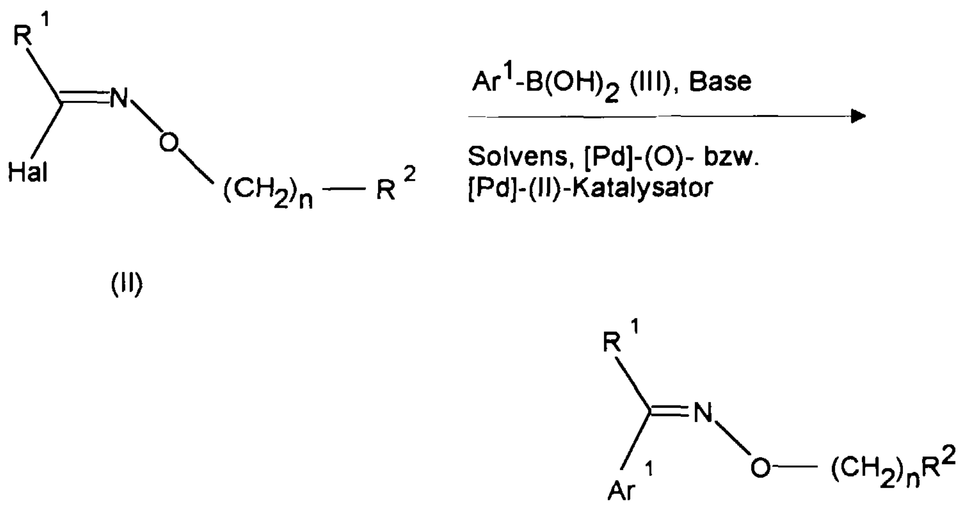


(II)

Schema 3

(I)

Der $Ar^1$-Rest der Boronsäure der Formel III tritt hierbei an die Stelle des Halogens Hal der Zwischenverbindung der Formel II.

Fluorhaltige Pflanzenschutzmittel, Arzneimittel und Substanzen für Materialwissenschaften gewinnen immer mehr an Bedeutung (Lt. J. T. Welch, Tetrahedron 43 (1987) 3123). Die Erzeugung von Reagentien für die Einführung von Halogenalkylgruppen, speziell Trifluormethyl bzw. die Herstellung fluorhaltiger Zwischenprodukte ist ein anderer Aspekt.

Mit der vorliegenden Erfindung werden erstmalig auch fluorhaltige Zwischenprodukte der Formel II ($R^1$ = Fluoralkyl) zur Verfügung gestellt, die neben dem Einsatz zur Herstellung von Pflanzenschutzmitteln der Formel I (z. B. insektizide Oximether WO-A-93/21157; Safener EP-A-94348; Fungizide EP-A-463488; Herbizide EP-A-461797) und Pharmazeutika (BE-A-862699) auch generell als Bausteine für organische Synthesen dienen können.

Besonders bevorzugtes Halogen Hal in den Zwischenprodukten der Formel II ist Chlor, $R^1$ bedeutet bevorzugt Trifluormethyl, n = 1, $Ar^1$ ein ein- oder zweifach beliebig substituiertes Aryl oder Heteroaryl wie Phenyl oder Pyridinyl, $R^2$ ein beliebig substituierter 5- oder 6-gliedriger ein- oder mehrkerniger Aromat oder Heteroaromat. Die daraus entstehenden Oximether der Formel I sind größtenteils aus der WO-A-93/21157 bekannt.

Bevorzugte Basen dieses Verfahrens sind anorganische Basen wie Ammoniak, Alkalimetalle, Alkali- und Erdalkalimetallhydride, Alkali- und Erdalkalimetallamide, Alkali- oder Erdalkalimetallalkoxide, Alkali- und Erdalkalimetallhydroxide, Alkali- und Erdalkalimetallhydrogencarbonate, Alkalimetallfluoride; bevorzugt aber Natriumcarbonat, Natriumhydrogencarbonat und Cäsiumfluorid in jeweils geeigneten Lösungsmitteln bzw. Lösungsmittelgemischen.

Geeignete Lösungsmittel dieses Verfahrens sind protische und/oder aprotische organische oder wäßrige Gemische verschiedener Lösungsmittel. Bevorzugte Lösungsmittel sind Alkohole wie Methanol, Ethanol, Propanol, Butanol; Ether wie Diethylether, Tetrahydrofuran, Ethylenglykolmonomethylether, Ethylenglykoldimethylether; Acetonitril, N-Methyl-pyrrolidon, Dimethylformamid, Dimethylacetamid, Dimethylsulfoxid; halogenierte aliphatische und aromatische Kohlenwasserstoffe wie Methylenchlorid, 1,2-Dichlorethan, Chlorbenzol; andere aromatische Lösungsmittel wie Toluol, Xylole oder Kresole.

Insbesondere bevorzugt sind alkoholische Lösungsmittel wie Methanol und Ethanol sowie n- und i-Propanol, aromatische Lösungsmittel wie Toluol oder Xylol und Polyalkylenglykoldialkylether wie 1,2-Dimethoxyethan und N-alkylierte Amide wie Dimethylacetamid sowie Gemische aus Toluol, oben genannten Alkoholen und Wasser bei Verwendung von Natriumcarbonat und Natriumhydrogencarbonat und 1,2-Dimethoxyethan bei Verwendung von Cäsiumfluorid.

Es ist in der Regel vorteilhaft, die Verbindung der Formel III im Verhältnis zur Verbindung der Formel II äquimolar oder im geringen Überschuß einzusetzen. Bevorzugt ist ein Molverhältnis für III:II von 1:1 bis 1:1,2.

Die Basen werden bevorzugt im Überschuß bezogen auf Verbindungen der Formel II eingesetzt, bevorzugt im Molverhältnis 1:2 bis 1:3.

Als Katalysatoren können unterschiedlichste Palladium-(0)- oder Palladium-(II)-Katalysatoren eingesetzt werden wie Palladium auf unterschiedlichsten Trägermaterialien wie Aktivkohle, Aluminiumoxid, Erdalkalimetallsulfaten u. ä., evtl. unter Zusatz von tert. Phosphinliganden wie beliebig substituierte Triarylphosphine, Alkylenbis-(tri/aryl)phosphine und andere in der Literatur bekannte Phosphinliganden. Andere geeignete Palladium-(O)- oder -(II)-Katalysatoren sind z. B. Tetrakis(triphenylphosphin)-palladium, Tris(dibenzylidenaceton)-dipalladium (=$Pd_2(dba)_3$); Bis(triphenylphosphin)-palladiumdichlorid, Palladium-(II)-[1,1'-bis-(diphenylphosphino)-ferrocenyl]chlorid und andere, in der folgenden Literatur beschriebene Palladiumkatalysatoren:

1) (a) Miyaura, N.; Yanagi, T.; Suzuki A., Synth. Commun. 1981, 11, 513. (b) Miller, R. B.; Dugar, S., Organometallics 1984, 3, 1261. (c) Thompson, W. J.; Gaudino, J., J. Org. Chem. 1984, 49, 5237. (d) Fu, J.-m.; Sharp, M. J.; Snieckus, V., Tetrahedron Lett. 1988, 29, 5459. (e) Sato, M.; Miyaura, N.; Suzuki, A., Chem. Lett. 1989, 1405. (f) Wallow, T. I.; Novak, B. M., J. Org. Chem. 1994, 59, 5034. (g) Huth, A.; Beetz, I.; Schumann, I., Tetrahedron 1989, 45, 6679. (h) Oh-e, T.; Miyaura, N., Suzuki, A., Synlett 1990, 221. (i) Fu, J.-m.; Snieckus, V., Tetrahedron Lett. 1990, 31, 1665. (j) Watanabe, T.; Miyaura, N; Suzuki, A., Synlett 1992, 207. (k) Oh-e, T.; Miyaura, N.; Suzuki, A., J. Org. Chem. 1993, 58, 2201. (l) Marck, G.; Villiger, A.; Buchecker, R., Tetrahedron Lett. 1994, 35, 3277.

2) (a) Martin, A. R.; Yang, Y., Acta Chem. Scand., 1993, 47, 221. (b) Ritter, K., Synthesis 1993, 735. (c) Cacchi, S. in Seminars in Organic Synthesis: XVIII Summer School "A. Corbella"; Polo Editoriale Chimico: Milan, Italy, 1993; p. 217. (d) Grushin, V. V.; Alper, H., Chem. Rev. 1994, 94, 1047. (e) Hegedus, L. S., J. Organomet. Chem. 1993, 457, 167.

3) Mitchell, M.B.; Wallbank, P.J., Tetrahedron Lett. 1991, 32, 2273.

Besonders bevorzugt sind Tetrakis(triphenylphosphin)-palladium, Pd/Aktivkohle (unterschiedliche Gehalte an Palladium) unter Zusatz von Triphenylphosphin in Molverhältnissen, bezogen auf Verbindungen der Formel II, von 0,01-5 %, bevorzugt 0,1 bis 2 Molprozent der Verbindung der allgemeinen Formel III.

Das erfindungsgemäße Verfahren wird vorzugsweise bei Temperaturen zwischen -30°C und Siedepunkt des jeweiligen Lösungsmittels bzw. Lösungsmittelgemisches durchgeführt; bevorzugte Temperaturen liegen, z. B. beim

Toluol/Ethanol/Wasser-Gemisch um 100°C.

Im allgemeinen wird bei Normaldruck gearbeitet; es ist jedoch möglich, in Abhängigkeit von den jeweiligen Reaktionsbedingungen, auch bei vermindertem bzw. erhöhtem Druck zu arbeiten.

Die Herstellung der Verbindungen der Formel III erfolgt nach literaturbekannten Verfahren (Houben-Weyl, Band XIII/3qa, S. 489 ff) oder in Analogie dazu.

Verbindungen der Formel II werden bevorzugt durch Halogenierung des jeweiligen Hydroxamsäureesters IV ($R^1$ = Halogenalkyl, Trifluormethyl) hergestellt.

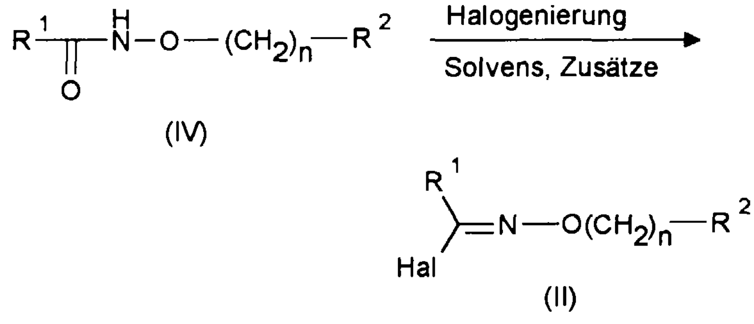

(IV)

(II)

Die für die Halogenierung geeigneten Halogenierungsmittel sind z.B. Phosphortrihalogenide, Phosphorpentahalogenide, Phosphoroxychlorid, Thionylchlorid, Phosgen, Triarylphosphin-Halogen-Addukte, Triarylphosphat-Halogen-Addukte, Triarylphosphin-Tetrahalogenkohlenstoff in Acetonitril, Cyanurchlorid und andere, in der Literatur beschriebene Chlorierungsmittel bei Temperaturen zwischen -30°C und Siedepunkt des jeweiligen Lösungsmittels (siehe auch S. Patai, The Chemistry of the Carbon-Nitrogen-Double bond, S. 607 ff., London 1970, Houben-Weyl Band E5, S. 631 ff. und H. Ulrich, The Chemistry of Imidoyl Halides, S. 1 ff., New York 1968).

Bevorzugte Halogenierungsmittel für Verbindungen der Formel IV (mit $n \neq 0$, vorzugsweise $n = 1$) und $R^2$ ist beliebig substituiertes Aryl und Heteroaryl sind Gemische aus Tetrahalogenkohlenstoff, Triarylphosphin in Acetonitril, speziell Tetrachlorkohlenstoff, Triphenylphosphin in Acetonitril bei Temperaturen zwischen 0 und 100, vorzugsweise 0 und 80 °C.

Die Hydroxamsäureester können nach literaturbekannten Verfahren zum einen durch Acylierung des entsprechenden Hydroxylamins (Houben-Weyl E5/628 ff., 82 ff.; A.S. Singha, B.N. Misra, J. Indian. Chem. Soc. 66 (1989) 101) bzw. durch Alkylierung der entsprechenden Hydroxamsäure (Houben-Weyl E5/826 ff.; W.J. Middleton, J. Org. Chem. 48 (1983) 3845) hergestellt werden.

Beispiel 1 (Tabellenbeispiel 7)

1-Chlor-2,2,2-trifluor-ethanon-O-benzyl-oxim

Unter Stickstoff werden 80,0 g (0,365 mol) N-Trifluoracetyl-O-benzylhydroxylamin (Fp. 58°C) in 600 ml Acetonitril bei Raumtemperatur vorgelegt. Anschließend wird in einer Portion 143,6 g (0,540 mol) Triphenylphosphin und 84,2 g (0,548 mol) Tetrachlorkohlenstoff dazugegeben und kräftig gerührt. Die Reaktionslösung erwärmt sich darauf auf 81°C. Nach Abkühlen auf Raumtemperatur ist die Reaktion i.a. beendet. Man engt im Vakuum (12 Torr, 40°C) ein und kocht den Kolbenrückstand mehrmals mit Petrolether (60/95°C) aus. Die gesammelten Filtrate werden eingeengt. Es verbleiben 65,2 g (72 %) eines leichtbeweglichen Öles (Z-Isomer). Evtl. kann das Produkt über Kieselgel mit einem Essigester-/Petrolethergemisch (1:4) gereinigt werden.

(Z)-Isomer

$^1$H-NMR (300 MHz, $CDCl_3$): δ (ppm) = 5,38 (s, 2H); 7,38 (m, 5H)
$^{19}$F-NMR (282,1 MHz, $CDCl_3$; $CFCl_3$): δ (ppm)= -69,3 (s)

Beispiel 2 (Tabellenbeispiel 30)

1-Chlor-2,2,2-trifluor-ethanon-O-(2-methyl-biphenyl-3-yl-methyl)-oxim

Unter Stickstoff werden 24,3 g (0,079 mol) N-Trifluoracetyl-O-2-methyl-3-phenyl)-benzyl-hydroxylamin (Fp. 83°C) in 200 ml Acetonitril vorgelegt und mit 30,9 g (0,118 mol) Triphenylphosphin und 18,13 g (0,178 mol) Tetrachlorkohlenstoff wie unter Beispiel 1 beschrieben, umgesetzt.

Ausbeute nach Kochen mit Petrolether: 19,5 g (76 %) eines gelben Öles.

(Z)-Isomer

$^1$H-NMR (300 MHz, $CDCl_3$): δ (ppm) = 2,25 (s, 3H); 5,4 (s, 2H); 7,2-7,45 (m, 8H)
$^{19}$F-NMR (282,1 MHz, $CFCl_3$; $CDCl_3$): δ (ppm) = -69,2 (s).

Beispiel 3 (Tabellenbeispiel 21)

1-Chlor-2,2,2-trifluor-ethanon-O-(2-brom-benzyl)-oxim

Analog Beispiel 1 werden 2,7 g (9,1 mmol) N-Trifluoracetyl-O-(2-brombenzylhydroxylamin) mit 3,54 g (13,5 mmol) Triphenylphosphin und 2,1 g (13,7 mmol) Tetrachlorkohlenstoff umgesetzt und aufgearbeitet.

Ausbeute: 59 % gelbes Öl.

(Z)-Isomer

$^1$H-NMR (300 MHz, $CDCl_3$): δ (ppm) = 5,39 (s, 2H); 7,40 (m, 4H)
$^{19}$F-NMR (282,1 MHz, $CDCl_3$; $CFCl_3$): δ (ppm) = -69,3 (s)

Beispiel 4 (Tabellenbeispiel 42)

1-Chlor-2,2,2-trifluor-ethanon-O-(pyridin-3-yl)-methyl-oxim

Analog Beispiel 1 werden 3,8 g (17,3 mmol) N-Trifluoracetyl-O-(3-pyridylmethyl)-hydroxyl-amin mit 6,8 g (25,9 mmol) Triphenylphosphin und 4,0 g (26,0 mmol) Tetrachlorkohlenstoff in 20 ml Acetonitril umgesetzt. Nach Entfernen des Lösungsmittels im Vakuum (12 Torr, 40°C) erhält man 2,79 g (68 %) zähes, gelbliches Öl ((Z)-Isomer).

$^1$H-NMR (300 MHz, $CDCl_3$): δ (ppm) = 5,5 (s, 2H); 8,8-7,8 (m, 4H)
$^{19}$F-NMR (282,1 MHz, $CDCl_3$; $CFCl_3$): δ (ppm) = -69,5 (s)

Beispiel 5

Phenyl-trifluormethyl-ketonoxim-O-benzylester Natriumhydrogencarbonatvariante

0,82 g (6,8 mmol) Phenylboronsäure wird mit 2,38 g (33,4 mmol) Natriumhydrogencarbonat, 0,24 g (0,106 mmol) Tetrakis(triphenylphosphin)palladium und 1,46 g (6,1 mmol) Chloroximinoverbindung des Tabellenbeispiels 7 in einem Gemisch aus 6,8 ml Toluol, 4,2 ml Ethanol und 14,45 ml Wasser auf 100°C erhitzt. Nach beendeter Reaktion wird im Vakuum (12 Torr/40°C) eingeengt, der Rückstand auf halbkonzentrierte Kochsalzlösung gegossen, 2 x mit Essigester extrahiert, die organische Phase über Natriumsulfat getrocknet.

| | |
|---|---|
| Rohausbeute: | 1,79 g |
| Isomerenverhältnis: | 95:5 = (E):(Z) |
| Reinausbeute: | 1,22 g (70 %) nach Chromatographie an 30 cm Kieselgel (Essigester:Petrolether = 1:20) |

Beispiel 6

3,6-Bis(trifluormethyl)phenyl-ketonoxim-O-(2-methyl-3-phenyl-phenyl)-methylether
Natriumhydrogencarbonat-Variante

Analog Patentbeispiel 5 werden 1,030 g (4,0 mmol) 3,6-Bis(trifluormethyl)phenylboronsäure, 1,40 g (16,7 mmol) Natriumhydrogencarbonat, 1,09 g (3,30 mmol) der Chlor-oximinoverbindung (Tabellenbeispiel 30) und 50,0 mg (0,04 mmol) Tetrakis(triphenylphosphin)palladium in einem Gemisch aus 8,0 ml Toluol, 3,6 ml Ethanol und 17,5 ml Wasser auf 100°C erhitzt. Nach 6 h (DC-Kontrolle) wird auf Raumtemperatur abgekühlt, im Vakuum (12 Torr, 40°C) eingeengt, mit halbkonz. Kochsalzlösung verdünnt und 2x mit Essigester extrahiert und über Natriumsulfat getrocknet.

Rohausbeute: 1,93 g (84 %)

Isomerenverhältnis: 88:12 = (E):(Z)

Nach Chromatographie über 50 cm Kieselgel (Petrolether:Essigester = 40:1) erhält man 1,42 g (62 %) eines farblosen Feststoffes (Fp. 72-77 °C) mit identischem Isomerenverhältnis.

$^{19}$F-NMR (282,1 MHz, $CDCl_3$, $CFCl_3$): δ (ppm) 66,3 (s, (E)); 62,6 (s, (Z)); 63,6 (s, 6F)

Beispiel 7 (Tabellenbeispiel 91)

(2-(4-Benzyloxyphenyl)-pyrimidin-5-yl)-trifluormethyl-ketonoxim-O-(2-methyl-3-phenyl-phenyl)-methyl-ether
Natriumcarbonat-Variante

0,76 g (2,33 mmol) Chloroximinoverbindung (Tabellenbeispiel 30), 0,85 g (2,79 mmol) 2-(4-Benzyloxyphenyl)-pyrimidin-5-boronsäure, 0,62 g (5,82 mmol) Natriumcarbonat und 30 mg (0,031 mmol) Tetrakis(triphenylphosphin)palladium werden in einem Gemisch aus 5,6 ml Toluol, 2,5 ml Ethanol und 12,2 ml Wasser 24 h zum Rückfluß erhitzt. Nach Abkühlen auf Raumtemperatur wird vom Ungelösten abfiltriert, im Vakuum eingeengt (12 Torr, 40°C), mit halbkonz. Kochsalzlösung verdünnt, 2x mit Essigester extrahiert und über Natriumsulfat getrocknet.

Rohausbeute: 1,1 g (86 %)
Isomerenverhältnis: 86:14 = (E):(Z)

Das Rohprodukt wurde über 30 cm Kieselgel (Essigester:Petrolether = 1:4) chromatographiert.

Ausbeute: 0,98 g (76 %)
Isomerenverhältnis: 86:14 = (E):(Z)

$^{19}$F-NMR (282,1 MHz, $CDCl_3$; $CFCl_3$): δ (ppm) = 66,6 (s; $CF_3C$=N, (E)); 63,6 (s, $CF_3C$=N, (Z)).

Beispiel 8

(4-Trifluormethoxyphenyl)-trifluormethyl-ketonoxim-O-(2-methyl-3-phenyl-phenyl)-methyl-ether
Cäsiumfluorid-Variante

Unter Stickstoff werden 208 mg (1,1 mmol) 4-Trifluormethoxyphenylboronsäure, 304 mg (2,2 mmol) Cäsiumfluorid, 326 mg (1,0 mmol) Chloroximinoverbindung (Tabellenbeispiel 30) sowie 38 mg (0,033 mmol) Tetrakis-(triphenylphosphin)-palladium in 10 ml Dimethoxyethan 24 h zum Rückfluß erhitzt (DC-Kontrolle). Anschließend wird auf Raumtemperatur abgekühlt, mit 0,1 g Aktivkohle versetzt und vom Ungelösten abfiltriert. Das so erhaltene Rohprodukt (15 cm, 95:5; 417 cm ≙ 92 %) wird anschließend über 30 cm Kieselgel (Essigester:Petrolether = 1:40) gereinigt.

Ausbeute: 376 mg (83 %) farbloses Öl
Isomerenverhältnis: (E):(Z) = 95:5

$^{19}$F-NMR (282,1 MHz, $CDCl_3$; $CFCl_3$) : δ (ppm) = -66,4 (s) (E); -62,6 (s) (Z); -58,2 (s)

Beispiel 9

(4-Trifluormethoxyphenyl)-trifluormethyl-ketonoxim-O-(2-methyl-3-phenyl-phenyl)-methyl-ether
Pd/C-Variante

Unter Stickstoff werden 1,09 g (3,34 mmol) Chloroximinoverbindung (Tabellenbeispiel 30) 0,83 g (4,0 mmol) 4-Trifluormethoxyphenylboronsäure, 50 mg (0,047 mmol) 10 % Pd/C, 50 mg (0,19 mmol) Triphenylphosphin und 0,88 g (8,35 mmol) Natriumcarbonat in einem Gemisch aus 8 ml Toluol, 3,6 ml Ethanol und 17,5 ml Wasser 24 h zum Rückfluß erhitzt (DC-Kontrolle). Nach Abkühlen auf Raumtemperatur wird vom Ungelösten abfiltriert, im Vakuum (12 Torr, 40°C) eingeengt, mit halbkonz. Kochsalzlösung verdünnt und 2x mit Essigester extrahiert und die organische Phase über Natriumsulfat getrocknet.

Rohausbeute: 1,51 g (100 %)
Isomerenverhältnis: 92:8 = (E):(Z)

Das Rohprodukt wurde anschließend durch Chromatographie auf 30 cm Kieselgel (Essigester:Petrolether = 1:20) gereinigt.

| | |
|---|---|
| Ausbeute: | 1,34 g (89 %) |
| Isomerenverhältnis: | 92:8 = (E):(Z) |

$^{19}$F (282,1 MHz, $CDCl_3$; $CFCl_3$) : δ (ppm) = -66,4 (s) (E); -62,6 (s) (Z); -58,2 (s)

Beispiel 10

(4-Trifluormethoxyphenyl)-trifluormethyl-ketonoxim-O-(2-methyl-3-phenyl-phenyl)-methyl-ether
Natriumcarbonat-Variante

Unter Stickstoff werden 5,45 g (16,7 mmol) Chloroximinoverbindung (Tabellenbeispiel 30) mit einem Gemisch aus 40 ml Toluol und 18 ml Ethanol vorgelegt. Diese Lösung wird anschließend mit 4,15 g (20,0 mmol) 4-Trifluormethoxyphenylboronsäure, einer Lösung aus 4,43 g (41,75 mmol) Natriumcarbonat in 87,5 ml Wasser und 0,25 g (220 mmol) Tetrakis(triphenylphosphin)-palladium 24 h zum Rückfluß erhitzt (DC-Kontrolle). Nach Abkühlen auf Raumtemperatur wird im Vakuum (12 Torr, 40°C) eingeengt, mit halbkonz. Kochsalzlösung verdünnt, 2x mit Essigester extrahiert und die organischen Phasen über Natriumsulfat getrocknet.

| | |
|---|---|
| Rohausbeute: | 8,2 g (100 %) |
| Isomerenverhältnis: | 90:10 = (E):(Z) |

Das so erhaltene Rohprodukt wurde anschließend über 50 cm Kieselgel (Essigester:Petrolether = 1:20) chromatographiert.

| | |
|---|---|
| Ausbeute: | 7,04 g (93 %) |
| Isomerenverhältnis: | 90:10 = (E):(Z) |

($^{19}$F-NMR s.o.)

Beispiel 11

(6-(2,2,2-Trifluorethoxy)pyridin-3-yl)-trifluormethyl-ketonoxim-O-(2-methyl-3-phenyl-phenyl)-methyl-ether
Natriumcarbonat-Variante

Unter Stickstoff werden 1,09 g (3,34 mmol) Chloroximinoverbindung (Tabellenbeispiel 30) mit 8 ml Toluol und 3,60 ml Ethanol versetzt. Zu dieser Lösung gibt man 0,88 g (4,0 mmol) 6-(2,2,2-Trifluorethoxypyridin)-3-boronsäure sowie eine Lösung aus 0,88 g (8,35 mmol) Natriumcarbonat in 17,5 ml Wasser und 50 mg (0,044 mmol) Tetrakis(triphenylphosphin)palladium. Die Mischung wird 24 h zum Rückfluß erhitzt (DC-Kontrolle). Anschließend wird auf Raumtemperatur abgekühlt, im Vakuum (12 Torr/40°C) eingeengt, mit halbkonzentrierter Kochsalzlösung verdünnt und 2x mit Essigester extrahiert.

| | |
|---|---|
| Rohausbeute: | 1,51 g (97 %) gelbes Öl |
| Isomerenverhältnis: | 94:6 = (E):(Z) |

Das Rohprodukt wurde über 30 cm Kieselgel (Essigester:Petrolether = 1:20) gereinigt.

Ausbeute: 1,34 g (86 %) farbloses Öl mit identischem Isomerenverhältnis

$^{19}$F-NMR (282,1 MHz, $CDCl_3$; $CFCl_3$): δ (ppm) = -66,9 (s) (E); -63,6 (s) (Z); -75,1 (t)

Die Verbindungen der nachfolgenden Tabelle wurden in analoger Weise hergestellt.

Tabelle

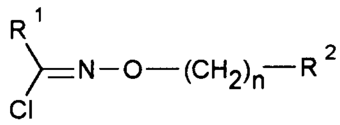

| Nr. | $R^1$ | n | $R^2$ | Stereochemie |
|---|---|---|---|---|
| 1 | $CF_3$ | 0 | $CH_3$ | (E) und (Z) |
| 2 | $CF_3$ | 1 | $CH_3$ | (E) und (Z) |
| 3 | $CF_3$ | 1 | $CH_2CH_3$ | (E) und (Z) |
| 4 | CF3 | 1 | Vinyl | (E) und (Z) |
| 5 | CF3 | 1 | 2,2-Dichlorvinyl | (E) und (Z) |
| 6 | CF3 | 1 | Ethinyl | (E) und (Z) |
| 7 | CF3 | 1 | Phenyl | (E) und (Z) |
| 8 | CF3 | 1 | 4-Fluorphenyl | (E) und (Z) |
| 9 | CF3 | 1 | 2,4-Difluorphenyl | (E) und (Z) |
| 10 | CF3 | 1 | 2,4,5-Trifluorphenyl | (E) und (Z) |
| 11 | CF3 | 1 | 2,3,5,6-Tetrafluor-4-methyl-phenyl | (E) und (Z) |
| 12 | CF3 | 1 | 2,3,5-Trifluor-6-methoxy-4-methyl-phenyl | (E) und (Z) |
| 13 | CF3 | 1 | 2,3,5,6-Tetrafluor-4-methoxy-phenyl | (E) und (Z) |
| 14 | CF3 | 1 | 2,6-Difluorphenyl | (E) und (Z) |
| 15 | CF3 | 1 | 2-Chlor-6-fluor-phenyl | (E) und (Z) |
| 16 | CF3 | 1 | 2,6-Difluor-4-trifluormethyl-phenyl | (E) und (Z) |
| 17 | CF3 | 1 | 4-Chlorphenyl | (E) und (Z) |
| 18 | CF3 | 1 | 2,4-Dichlorphenyl | (E) und (Z) |
| 19 | CF3 | 1 | 2,4,5-Trichlorphenyl | (E) und (Z) |
| 20 | CF3 | 1 | 4-Brom-phenyl | (E) und (Z) |

| Nr. | $R^1$ | n | $R^2$ | Stereochemie |
|---|---|---|---|---|
| 21 | CF3 | 1 | 2-Bromphenyl | (E) und (Z) |
| 22 | CF3 | 1 | 4-Trifluormethylphenyl | (E) und (Z) |
| 23 | CF3 | 1 | 2,6-Di-(trifluor-methyl)-phenyl | (E) und (Z) |
| 24 | CF3 | 1 | 4-Methylphenyl | (E) und (Z) |
| 25 | CF3 | 1 | 3-Methylphenyl | (E) und (Z) |
| 26 | CF3 | 1 | 2-Methylphenyl | (E) und (Z) |
| 27 | CF3 | 1 | 3,4-Dimethyl-phenyl | (E) und (Z) |
| 28 | CF3 | 1 | Biphenyl-4-yl | (E) und (Z) |
| 29 | CF3 | 1 | Biphenyl-3-yl | (E) und (Z) |
| 30 | CF3 | 1 | 2-Methyl-biphenyl-3-yl | (E) und (Z) |
| 31 | CF3 | 1 | 4-Phenoxyphenyl | (E) und (Z) |
| 32 | CF3 | 1 | 3-Phenoxyphenyl | (E) und (Z) |
| 33 | CF3 | 1 | 4-Fluor-3-phenoxyphenyl | (E) und (Z) |
| 34 | CF3 | 1 | 4-Methoxyphenyl | (E) und (Z) |
| 35 | CF3 | 1 | 4-Trifluormethoxyphenyl | (E) und (Z) |
| 36 | CF3 | 1 | 4-Nitrophenyl | (E) und (Z) |
| 37 | CF3 | 1 | 4-Dimethylaminophenyl | (E) und (Z) |
| 38 | CF3 | 1 | 2-Naphthyl | (E) und (Z) |
| 39 | CF3 | 1 | 1-Naphthyl | (E) und (Z) |
| 40 | CF3 | 1 | 2-Thienyl | (E) und (Z) |
| 41 | CF3 | 1 | 2-Pyridyl | (E) und (Z) |
| 42 | CF3 | 1 | 3-Pyridyl | (E) und (Z) |
| 43 | CF3 | 1 | 4-Pyridyl | (E) und (Z) |
| 44 | CF3 | 1 | 6-Phenoxypyridin-2-yl | (E) und (Z) |
| 45 | CF3 | 1 | 6-Chlorpyridin-3-yl | (E) und (Z) |
| 46 | CF3 | 1 | 2-Ethoxypyrimidin-5-yl | (E) und (Z) |
| 47 | CF3 | 1 | 3-Methyl-3H-imidazol-4-yl | (E) und (Z) |

| Nr. | $R^1$ | n | $R^2$ | Stereochemie |
|---|---|---|---|---|
| 48 | CF3 | 1 | Benzofuran-6-yl | (E) und (Z) |
| 49 | CF3 | 1 | Benzo[1,3]dioxol-5-yl | (E) und (Z) |
| 50 | CF3 | 1 | 2-Furyl | (E) und (Z) |
| 51 | $CF_2CF_3$ | 1 | Phenyl | (E) und (Z) |
| 52 | $CF_2CF_3$ | 1 | Biphenyl-4-yl | (E) und(Z) |
| 53 | $CF_2$CF3 | 1 | 2-Methyl-biphenyl-3-yl | (E) und (Z) |
| 54 | $CF_2$CF3 | 1 | 2,3,5,6-Tetrafluor-4-methylphenyl | (E) und (Z) |
| 55 | $CF_2$CF3 | 1 | 2,3,5,6-Tetrafluor-4-methoxyphenyl | (E) und (Z) |
| 56 | $CF_2$CF3 | 1 | 3-Phenoxyphenyl | (E) und (Z) |
| 57 | $CF_2$CF3 | 1 | 4-Fluor-3-phenoxyphenyl | (E) und (Z) |
| 58 | $CF_3$ | 0 | Phenyl | (E) und (Z) |
| 59 | $CF_3$ | 0 | 4-Chlorphenyl | (E) und (Z) |
| 60 | CF3 | 0 | 2,6-Dichlor-4-trifluormethylphenyl | (E) und (Z) |
| 61 | CF3 | 0 | 4-Formylphenyl | (E) und (Z) |
| 62 | CF3 | 0 | 2-Pyridyl | (E) und (Z) |
| 63 | CF3 | 0 | 4-Pyridyl | (E) und (Z) |
| 64 | CF3 | 0 | Pyrimidin-2-yl | (E) und (Z) |

## Patentansprüche

**1.** Verfahren zur Herstellung einer Verbindung der Formel (I)

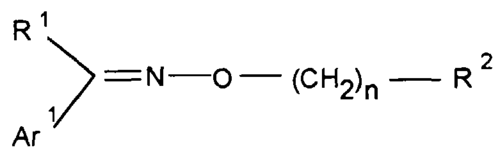

(I),

in welcher

- $Ar^1$ einen gegebenenfalls substituierten fünf- oder sechsgliedrigen monocyclischen oder anellierten aromatischen oder heteroaromatischen Rest bedeutet;
- $R^1$ einen gegebenenfalls substituierten Alkyl-, Cycloalkyl-, Alkenyl- oder Alkinyl-Rest bedeutet,
- $R^2$ wie $Ar^1$ oder $R^1$ definiert ist; und
- n eine ganze Zahl von 1 bis 10 ist,

dadurch gekennzeichnet, daß man eine Verbindung der Formel II

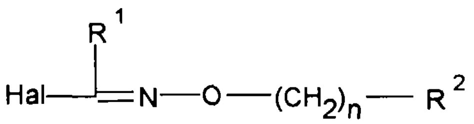

(II),

in welcher $R^1$, $R^2$ und n wie oben definiert sind, Hal, Fluor, Chlor, Brom oder Iod bedeutet, mit einer Boronsäure der Formel III

$$Ar^1—B(OH)_2 \qquad (III),$$

in welcher $Ar^1$ wie oben substituiert ist, oder einem Dimeren oder Trimeren dieser Verbindung in Gegenwart einer Palladium-(O)- oder Palladium-(II)-Verbindung und einer Base in einem organischen Lösungsmittel oder Lösungsmittelgemisch umsetzt.

**2.** Verfahren gemäß Anspruch 1, bei welchem eine Verbindung der Formel I hergestellt wird, in welcher

I.

$Ar^1$

a) ($C_6$-$C_{12}$)-Aryl oder Heteroaryl mit bis zu 10 C-Atomen bedeutet oder
b) wie unter I.a) definiert ist und bis zu 5 gleiche oder verschiedene Substituenten trägt aus der Reihe

1. ($C_1$-$C_6$)-Alkyl,
2. ($C_2$-$C_6$)-Alkenyl,
3. ($C_2$-$C_6$)-Alkinyl,
4. ($C_3$-$C_8$)-Cycloalkyl, gegebenenfalls substituiert mit bis zu 6 gleichen oder verschiedenen Resten aus der Reihe Halogen und ($C_1$-$C_4$)-Alkyl,
5. Halogen,
6. ($C_1$-$C_6$)-Halogenalkyl,
7. ($C_2$-$C_6$)-Halogenalkenyl,
8. ($C_2$-$C_6$)-Halogenalkinyl,
9. ($C_1$-$C_6$)-Alkoxy-($C_1$-$C_6$)-alkyl,
10. ($C_6$-$C_{12}$)-Aryl, gegebenenfalls bis zu dreifach substituiert mit gleichen oder verschiedenen Resten aus der Reihe ($C_1$-$C_6$)-Alkyl, ($C_1$-$C_6$)-Alkoxy, Halogen, ($C_1$-$C_6$)-Halogenalkyl und ($C_1$-$C_6$)-Halogenalkoxy,
11. Heteroaryl mit bis zu 10 C-Atomen, gegebenenfalls substituiert wie unter I. b) 10.,
12. ($C_6$-$C_{12}$)-Aryl-($C_1$-$C_4$)-alkyl, im Arylteil gegebenenfalls substituiert wie unter I. b) 10.,
13. Heteroaryl-($C_1$-$C_4$)-alkyl mit bis zu 10 C-Atomen im Heteroarylteil und dort gegebenenfalls substituiert wie unter I. b) 10.,
14. ($C_1$-$C_6$)-Alkoxy,
15. ($C_2$-$C_6$)-Alkenyloxy,
16. ($C_2$-$C_6$)-Alkinyloxy,
17. ($C_3$-$C_8$)-Cycloalkyloxy, gegebenenfalls substituiert wie unter I. b) 4.,
18. ($C_1$-$C_6$)-Alkoxy-($C_1$-$C_4$)-alkoxy,
19. ($C_6$-$C_{12}$)-Aryloxy, gegebenenfalls substituiert wie unter I. b) 10.,
20. Heteroaryloxy mit bis zu 10 C-Atomen, gegebenenfalls substituiert wie unter I. b) 10.,
21. ($C_1$-$C_6$)-Halogenalkoxy,
22. ($C_2$-$C_6$)-Halogenalkenyloxy,
23. ($C_2$-$C_6$)-Halogenalkinyloxy,
24. -O-N=CR'$_2$, worin R' für gleiche oder verschiedene Reste aus der Reihe Wasserstoff, ($C_1$-$C_6$)-Alkyl, ($C_3$-$C_8$)-Cycloalkyl und ($C_6$-$C_{12}$)-Aryl steht,
25. ($C_1$-$C_6$)-Alkylamino,
26. Di-($C_1$-$C_6$)-alkylamino,
27. ($C_3$-$C_8$)-Cycloalkylamino, gegebenenfalls substituiert wie unter I.b) 4.,

28. $(C_6-C_{12})$-Arylamino, gegebenenfalls substituiert wie unter I. b) 10.,
29. Heteroarylamino mit bis zu 10 C-Atomen, gegebenenfalls substituiert wie unter I. b) 10.,
30. $(C_1-C_6)$-Alkylmercapto,
31. $(C_6-C_{12})$-Arylmercapto,
32. Heteroarylmercapto mit bis zu 10 C-Atomen,
33. $(C_1-C_6)$-Alkylsulfinyl,
34. $(C_6-C_{12})$-Arylsulfinyl,
35. Heteroarylsulfinyl mit bis zu 10 C-Atomen,
36. $(C_1-C_6)$-Alkylsulfonyl,
37. $(C_6-C_{12})$-Arylsulfonyl,
38. Heteroarylsulfonyl mit bis zu 10 C-Atomen,
39. Nitro,
40. Cyano,
41. Cyano-$(C_1-C_6)$-alkyl,
42. $(C_1-C_6)$-Alkoxycarbonyl,
43. $(C_6-C_{12})$-Aryloxycarbonyl und
44. Heteroaryloxycarbonyl mit bis zu 10 C-Atomen im Heteroarylteil, oder
45.

a) zwei der Substituenten für Methylendioxy stehen,
b) wobei die Methylendioxygruppe gegebenenfalls mit einem oder zwei gleichen oder verschiedenen Resten aus der Reihe Halogen und $(C_1-C_4)$-Alkyl substituiert ist;

II.

$R^1$

a) $(C_1-C_6)$-Alkyl,
b) $(C_2-C_6)$-Alkenyl,
c) $(C_2-C_6)$-Alkinyl oder
d) $(C_3-C_8)$-Cycloalkyl bedeutet, oder
e) wie unter II. a)-d) definiert ist und bis zu 3 gleiche oder verschiedene Substituenten trägt aus der Reihe

1. Halogen,
2. Hydroxy,
3. $(C_1-C_6)$-Alkoxy,
4. $(C_1-C_6)$-Alkylmercapto,
5. $(C_1-C_6)$-Alkylsulfinyl,
6. $(C_1-C_6)$-Alkylsulfonyl,
7. Cyano,
8. Nitro und
9. $(C_1-C_6)$-Alkoxycarbonyl, oder

f) wie unter II. a)-d) definiert ist und, falls die Anzahl der Wasserstoffatome $\geq 5$ ist, diese teilweise oder vollständig durch Halogen ersetzt sind, wobei die Anzahl der Halogenatome $\geq 4$ ist;

wobei das unter I. a) und b) definierte Aryl und Heteroaryl auch teilweise hydriert sein kann und darin eine oder zwei $CH_2$-Gruppen durch CO ersetzt sein können.

**3.** Verfahren gemäß Anspruch 1 oder 2, bei welchem eine Verbindung der Formel I hergestellt wird, in welcher

$Ar^1$ Phenyl, Naphthyl, Indanyl, Benzofuryl, Benzothienyl,

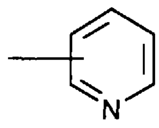
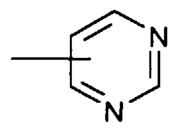
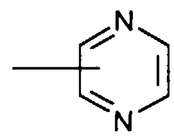
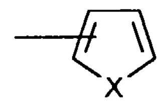

wie Thienyl oder Furanyl oder

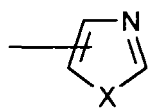 wie Thiazolyl bedeutet,

welche gegebenenfalls wie oben definiert substituiert sind und worin

X für O, S oder $NR^3$ steht und

$R^3$ für Wasserstoff,
($C_1$-$C_6$)-Alkyl,
($C_2$-$C_6$)Alkenyl,
($C_2$-$C_6$)-Alkinyl,
($C_3$-$C_8$)-Cycloalkyl, gegebenenfalls substituiert mit bis zu 6 gleichen oder verschiedenen Resten aus der Reihe Halogen und ($C_1$-$C_4$)-Alkyl,
($C_1$-$C_6$)-Halogenalkyl,
($C_2$-$C_6$)-Halogenalkenyl,
($C_2$-$C_6$)-Halogenalkinyl,
($C_1$-$C_6$)-Alkoxy-($C_1$-$C_6$)-alkyl,
($C_6$-$C_{12}$)-Aryl-($C_1$-$C_4$)-alkyl,
Heteroaryl-($C_1$-$C_4$)-alkyl mit bis zu 10 C-Atomen im Heteroarylteil,
Cyano-($C_1$-$C_6$)-alkyl,
($C_1$-$C_6$)-Alkoxycarbonyl,
($C_6$-$C_{12}$)-Aryloxycarbonyl oder
Heteroaryloxycarbonyl mit bis zu 10 C-Atomen steht.

4. Verfahren gemäß einem der Ansprüche 1 bis 3, bei welchem eine Verbindung der Formel I hergestellt wird, in welcher

$Ar^1$ einen Rest der Formel

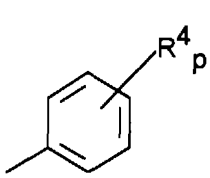
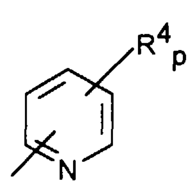
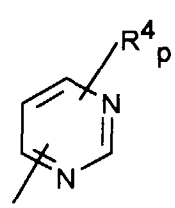
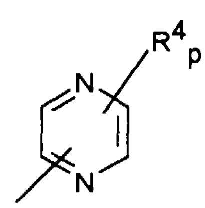

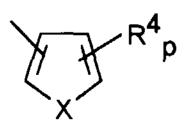 oder 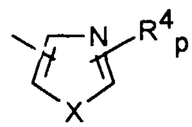

bedeutet,

X wie oben definiert ist,

p eine ganze Zahl von 0 bis 5 ist, und

$R^1$ gleich oder verschieden ist und
Halogen,
$(C_1-C_6)$-Alkyl,
$(C_2-C_6)$-Alkenyl,
$(C_2-C_6)$-Alkinyl,
$(C_3-C_8)$-Cycloalkyl, gegebenenfalls substituiert mit bis zu 6 gleichen oder verschiedenen Resten aus der Reihe Halogen und $(C_1-C_4)$-Alkyl,
$(C_1-C_6)$-Halogenalkyl,
$(C_2-C_6)$-Halogenalkenyl,
$(C_2-C_6)$-Halogenalkinyl,
$(C_1-C_6)$-Alkoxy-$(C_1-C_6)$-alkyl,
$(C_6-C_{12})$-Aryl, gegebenenfalls bis zu dreifach substituiert mit gleichen oder verschiedenen Resten aus der Reihe Halogen, $(C_1-C_6)$-Alkyl, $(C_1-C_6)$-Alkoxy, $(C_1-C_6)$-Halogenalkyl und $(C_1-C_6)$-Halogenalkoxy,
Heteroaryl mit bis zu 10 C-Atomen, gegebenenfalls substituiert wie obengenanntes Aryl,
$(C_6-C_{12})$-Aryl-$(C_1-C_4)$-alkyl, im Arylteil gegebenenfalls substituiert wie obengenanntes Aryl,
Heteroaryl-$(C_1-C_4)$-alkyl mit bis zu 10 C-Atomen im Heteroarylteil und dort gegebenenfalls substituiert wie obengenanntes Aryl,
$(C_1-C_6)$-Alkoxy,
$(C_2-C_6)$-Alkenyloxy,
$(C_2-C_6)$-Alkinyloxy,
$(C_3-C_8)$-Cycloalkyloxy,
$(C_1-C_6)$-Alkoxy-$(C_1-C_6)$-alkoxy,
$(C_6-C_{12})$-Aryloxy, gegebenenfalls substituiert wie obengenanntes Aryl,
Heteroaryloxy mit bis zu 10 C-Atomen, gegebenenfalls substituiert wie obengenanntes Aryl,
$(C_1-C_6)$-Halogenalkoxy,
$(C_2-C_6)$-Halogenalkenyloxy,
$(C_2-C_6)$-Halogenalkinyloxy oder
-O-N=CR'$_2$, worin R' gleiche oder verschiedene Reste aus der Reihe Wasserstoff, $(C_1-C_6)$-Alkyl, $(C_3-C_8)$-Cycloalkyl und $(C_6-C_{12})$-Aryl steht,
bedeutet oder
zwei der Reste für $R^4$ für gegebenenfalls wie unter I. b) 45. b)
substituiertes Methylendioxy stehen.

**5.** Verfahren gemäß einem der Ansprüche 1 bis 4, bei welchem eine Verbindung der Formel I hergestellt wird, in welcher

$R^1$ vorzugsweise $(C_1-C_6)$-Alkyl oder $(C_3-C_8)$-Cycloalkyl, welche jeweils teilweise oder vollständig mit Halogen substituiert sein können, bedeutet.

**6.** Verfahren gemäß einem der Ansprüche 1 bis 5, bei welchem eine Verbindung der Formel I hergestellt wird, in welcher

$R^2$

A.

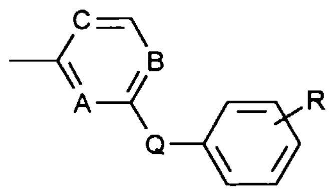

B. a) Pentafluorphenyl,

b) 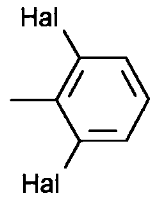

c) 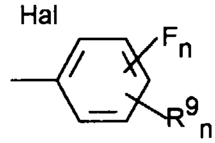

d) 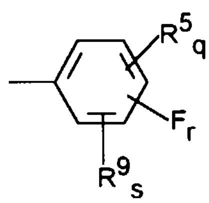

C.

a) 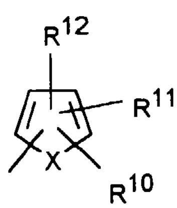

X = O oder S

b) 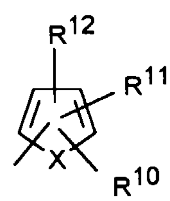

X = $NR^3$

c) 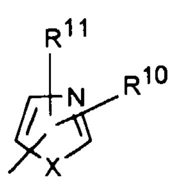

X = O, S oder $NR^3$

d) 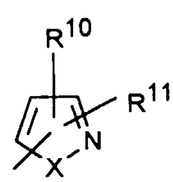

X = O, S oder $NR^3$

D. 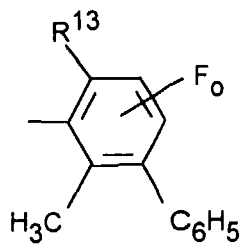

E. 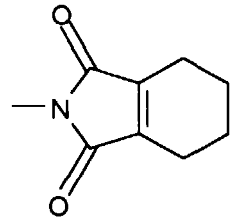

F.
a) 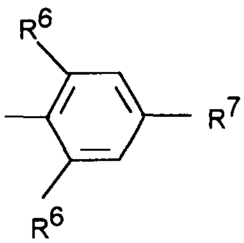

oder

b) 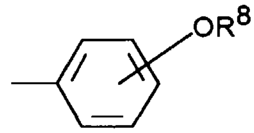

bedeutet, und worin

| | |
|---|---|
| A, B und C | gleich und verschieden sind und N, CH oder C-Hal bedeuten, |
| Q | O, S, NH oder $CH_2$, vorzugsweise O oder $CH_2$ bedeutet, |
| R | Wasserstoff, Halogen, O-$CH_2$-CH=$CH_2$ oder O-$CH_2$-C≡CH, vorzugsweise Wasserstoff, 4-F, 3-F, 4-Cl |
| Hal | für Halogen steht, |
| $R^9$ | Wasserstoff bedeutet oder wie $R^4$ definiert ist, |
| m und n' | gleich oder verschieden sind und jeweils für 1, 2, 3 oder 4 stehen, $m + n' = 5$ ist, |
| $R^5$ | Wasserstoff bedeutet oder wie $R^4$ definiert ist, |
| q, r und s | gleich oder verschieden sind und jeweils für 1, 2 oder 3 stehen, $q + r + s = 5$ ist, |
| $R^3$ | wie oben definiert ist, |
| $R^{10}$, $R^{11}$ und $R^{12}$ | wie oben definiert sind, vorzugsweise $CH_3$, $CF_3$, CN, Phenyl, $CH_2$-CH=$CH_2$, -$CH_2$-C≡CH oder Benzyl bedeuten, |
| $R^{13}$ | Wasserstoff, Fluor oder Methyl bedeutet, |
| o | 0, 1 oder 2 ist, |
| $R^6$ | Wasserstoff, Fluor oder ($C_1$-$C_6$)-Alkyl bedeutet, |
| $R^7$ | Halogenphenoxy, Halogenbenzyl, O-$CH_2$-CH=$CH_2$, O-$CH_2$-C≡CH, $CH_2$-CH=$CH_2$ oder $CH_2$-C≡CH bedeutet und |
| $R^8$ | ($C_2$-$C_6$)-Alkenyl oder ($C_2$-$C_6$)-Halogenalkenyl bedeutet. |

**7.** Verbindung der Formel II

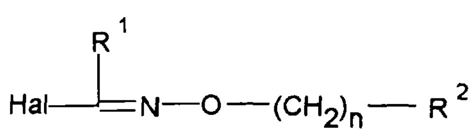

(II),

in welcher $R^1$, $R^2$, Hal und n wie in einem der Ansprüche 1 bis 6 definiert sind.

**8.** Verfahren zur Herstellung einer Verbindung der Formel II gemäß Anspruch 7, dadurch gekennzeichnet, daß man einen Hydroxamsäureester der Formel IV

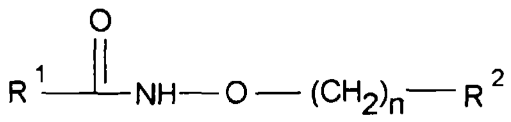

(IV),

in welcher $R^1$, $R^2$ und n wie in einem der Ansprüche 1 bis 6 definiert sind, halogeniert.

Europäisches Patentamt

# EUROPÄISCHER RECHERCHENBERICHT

Nummer der Anmeldung
EP 97 10 2048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | WO 95 20569 A (CIBA GEIGY AG ;ZIEGLER HUGO (CH); NEFF DENIS (CH); STUTZ WOLFGANG) 3.August 1995<br>* Ansprüche; Beispiele 1,4 * | 1,8 | C07C249/12<br>C07C251/34<br>C07C251/50<br>C07C251/38<br>C07C251/52 |
| X | INDIAN JOURNAL OF CHEMISTRY, Bd. 23b, 1984, Seiten 728-32, XP000672806<br>B. N. MISRA ET AL.: "Reactions of N-acyl-O-alkylhydroxylamines: Part V - Preparations of hydroxamic ester chlorides & their nucleophilic reactions"<br>* Seite 730, rechte Spalte, Zeile 7 - Seite 731, linke Spalte, Zeile 18 * | 8 | |
| X | TETRAHEDRON, Bd. 52, Nr. 1, 1.Januar 1996, OXFORD GB, Seiten 233-244, XP002031942<br>TAKASHI SAKAI ET AL.: "Asymmetric reduction of 2-(N-arylimino)-3,3,3-trifluoropropanoic acid esters leading to enantiomerically enriched 3,3,3-trifluoroalanine"<br>* Seite 240, Zeile 7 - Zeile 15 * | 7,8 | **RECHERCHIERTE SACHGEBIETE (Int.Cl.6)**<br>C07C |
| X | JOURNAL OF ORGANIC CHEMISTRY, Bd. 50, Nr. 7, 1985, EASTON US, Seiten 993-97, XP002031944<br>J. E. JOHNSON ET AL.: "Synthesis and configuration of O-substituted hydroximoyl chlorides. Stereochemistry and mechanism of alkoxide ion substitution at the carbon-nitrogen double bond"<br>* Seite 994, linke Spalte, Zeile 36 - rechte Spalte, Zeile 5 *<br>* Seite 996, linke Spalte, letzter Absatz - rechte Spalte, Zeile 23 *<br>-/-- | 7,8 | |

Der vorliegende Recherchenbericht wurde für alle Patentansprüche erstellt

| Recherchenort | Abschlußdatum der Recherche | Prüfer |
|---|---|---|
| DEN HAAG | 30.Mai 1997 | Seufert, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 10 2048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | CHEMICAL ABSTRACTS, vol. 73, no. 7, 17.August 1970<br>Columbus, Ohio, US;<br>abstract no. 34750,<br>G. KAMAI ET AL.: "Bromination of some O-derivatives of aldoximes"<br>Seite 279;<br>XP002031946<br>* Zusammenfassung *<br>& IZV. VYSSH. UCHEB. ZAVED., KHIM. KHIM. TEKHNOL.,<br>Bd. 13, Nr. 1, 1970,<br>Seiten 227-9,<br>--- | 7 | |
| X | US 5 328 906 A (LITERATI NAGY PETER ET AL) 12.Juli 1994<br>* Beispiele 7,8,14 *<br>--- | 7 | |
| X | JOURNAL OF ORGANIC CHEMISTRY,<br>Bd. 59, Nr. 4, 1994, EASTON US,<br>Seiten 929-31, XP002031945<br>TAKESHI SAKAMOTO ET AL.: "A new synthesis of N.alpha., N.delta.-protected N.delta. Hydroxycycloornithine and its homologue from a L-glutamic acid derivative"<br>* Seite 930, Verbindung 7 *<br>---<br>-/-- | 7 | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| DEN HAAG | 30.Mai 1997 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 10 2048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | CHEMICAL ABSTRACTS, vol. 120, no. 1, 3.Januar 1994 Columbus, Ohio, US; abstract no. 7991, G. SHUSTOV ET AL.: "Ketenimine-nitrile rearrangement of N-alkoxyketenimines under conditions of their formation" Seite 884; XP002031947 * Zusammenfassung * & IZV. AKAD. NAUK, SER. KHIM., Nr. 11, 1992, Seiten 2584-91, --- | 7 | |
| X | CHEMICAL ABSTRACTS, vol. 101, no. 1, 2.Juli 1984 Columbus, Ohio, US; abstract no. 2319, "Compositions containing oxime derivatives and their use in the protection of cultivated plants" Seite 208; XP002031948 * Zusammenfassung * & IL 55 470 A (CIBA-GEIGY) --- | 7 | RECHERCHIERTE SACHGEBIETE (Int.Cl.6) |
| X | DE 27 16 677 A (HOECHST AG) 26.Oktober 1978 * Seite 164, Zeile 12 - Zeile 15 * * Seite 158, Zeile 14 - Zeile 24 * --- | 7 | |
| X | DATABASE CROSSFIRE Beilsteininformationssysteme GmbH XP002031949 & CHEM. BER., Bd. 22, 1889, Seite 2397 --- -/-- | 7 | |

Der vorliegende Recherchenbericht wurde für alle Patentansprüche erstellt

| Recherchenort | Abschlußdatum der Recherche | Prüfer |
|---|---|---|
| DEN HAAG | 30.Mai 1997 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 10 2048

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| P,X | JOURNAL OF THE AMERICAN CHEMICAL SOCIETY, Bd. 118, Nr. 21, 29.Mai 1996, DC US, Seiten 5138-39, XP002031943 SUNGGAK KIM ET AL.: "Novel radical reaction of phenylsulfonyl oxime ethers. A free radical acylation approach" * Fussnote 16) * ----- | 7 | |
| | | | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| DEN HAAG | 30.Mai 1997 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)